# EUROPÄISCHE PATENTSCHRIFT

(11) **EP 1 433 411 B1**
(45) Veröffentlichungstag und Bekanntmachung des Hinweises auf die Patenterteilung: **16.01.2008**
(21) Anmeldenummer: 04006220.0
(22) Anmeldetag: 23.03.1998
(51) Int. Cl.: A61B 1/01, A61B 1/31

(54) **Stülpschlauchsystem**
Everting sleeve system
Système à manchon inversé

(30) Priorität: 23.04.1997 DE 19717108
(43) Veröffentlichungstag der Anmeldung: 30.06.2004
(62) Teilanmeldung aus: 98105235.0
(73) Patentinhaber: invendo medical GmbH, 69469 Weinheim (DE)
(72) Erfinder: Viebach, Thomas, 86579 Diepoltshofen (DE); Pauker, Fritz, 86316 Wiffertshausen/Friedberg (DE); Buchmann, Gerhard, 82335 Berg (DE); Weiglhofer, Gerhard, 86947 Schwabhausen/Weil (DE); Pauker, Robert, 86438 Kissing (DE)
(74) Vertreter: TBK-Patent

(56) Entgegenhaltungen:
- DE-A- 3 925 484
- DE-A- 4 242 291
- US-A- 4 211 233

## Beschreibung

Die Erfindung betrifft ein Endoskop gemäß Patentanspruchs 1.

Aus dem Stand der Technik beispielsweise gemäß der DE 42 42 291 A1 ist nunmehr ein Endoskop dieser Gattung bekannt.

Dieses Endoskop besteht im wesentlichen aus einem Endoskopkopf oder distalen Ende, an das sich ein Endoskopschaft aus einem flexiblen biegsamen Rohrkörper anschließt und einer Bedienungseinrichtung am hinteren Ende des Endoskopschafts.

Um den Endoskopschaft ist zumindest in dessen vorderem Abschnitt ein Stülpschlauch angeordnet, der durch eine Antriebs- oder Vorschubeinrichtung angetrieben wird. Der Stülpschlauch besteht dabei aus einem inneren Schlauchabschnitt, der gleitfähig an der Mantelfläche des Endoskopschafts anliegt und im Bereich des distalen Endes des Endoskops zu einem vorderen äußeren Schlauchabschnitt umgestülpt ist. Der vordere äußere Schlauchabschnitt ist ferner bis zur Antriebseinrichtung zurückgeführt und an deren Gehäuse fixiert. Im hinteren Bereich des Endoskops ist der innere Schlauchabschnitt zu einem hinteren äußeren Schlauchabschnitt umgestülpt, der ebenfalls zur Antriebseinrichtung zugeführt und an deren Gehäuse an der zum vorderen äußeren Schlauchabschnitt gegenüberliegenden axialen Endseite fixiert ist.

Da bei dieser alleinigen Antriebsart durch die Antriebseinrichtung die Vorschubgeschwindigkeit (und Strecke) des Stülpschlauchs an seinem vorderen Stülpbereich aufgrund dessen Stülpbewegung nur halb so groß wäre, wie die des Endoskopschafts, d.h. der Endoskopschaft würde mit zunehmender Eindringtiefe aus dem Stülpschlauch teleskopförmig herausfahren, übt eine weitere Antriebseinrichtung eine Bremskraft auf den Endoskopschaft aus, die der Vorschubkraft der zweiten Antriebseinrichtung entgegenwirkt.

Die Antriebseinrichtung ist dabei zu der Bremseinrichtung synchronisiert, derart, daß im Zusammenspiel beider Einrichtungen die Bewegungsgeschwindigkeit des inneren Schlauchabschnitts in eine axiale Richtung doppelt so hoch ist wie die Bewegungsgeschwindigkeit des Endoskopschafts, wobei dieser relativ zum inneren Endoskopschaft gleitet (d.h. das distale Ende des Endoskopschafts bewegt sich mit gleicher Geschwindigkeit wie der Umstülpbereich des Stülpschlauchs).

Um die Relativgleitbewegung zwischen dem Endoskopschaft und dem Stülpschlauch zu erleichtern, sieht der Stand der Technik gemäß der DE 42 42 291 A1 ferner eine Schmiervorrichtung vor, mittels der ein Schmier- oder Gleitmittel zwischen den inneren Schlauchabschnitt und den Endoskopschaft sowie zwischen den inneren und äußeren Schlauchabschnitt einpreßbar ist. Hierfür hat die Schmiervorrichtung eine konusförmige Hülse, die über den Endoskopschaft gestreift ist und mit dem hinteren Stülpbereich des Stülpschlauchs, der auf die konusförmige Hülse aufreitet, dichtend zusammenwirkt. Das Schmiermittel, welches mittels einer Pumpe in den Spalt zwischen der konusförmigen Hülse und dem Endoskopschaft eingepreßt wird, breitet sich zwischen dem inneren Schlauchabschnitt und dem Endoskopschaft auf die gesamte Länge aus, wobei Überschußmengen des Schmiermittels im vorderen Umstülpbereich des Stülpschlauchs in den zu untersuchenden Hohlraum austreten.

Es hat sich nunmehr gezeigt, daß das Endoskop gemäß der DE 42 42 291 A1 insbesondere hinsichtlich des Antriebs des Stülpschlauchs sowie des Endoskopschafts als auch der relativ hohen Leckageverluste an Schmiermittel im vorderen Umstülpbereich des Stülpschlauchs gewisse Mängel aufweist, die den Einsatz des Endoskops erschweren.

Es ist daher die Aufgabe der vorliegenden Erfindung, ein Endoskop mit Stülpschlauchsystem derart weiterzubilden, daß dessen Funktionalität und Handhabung verbessert wird.

Diese Aufgabe wird erfindungsgemäß durch ein Endoskop mit den Merkmalen des Anspruchs 1 gelöst.

Als weisentlicher Aspekt der Erfindung ist es nach Anspruch 1 beabsichtigt, das Endoskop mit einer Schmiervorrichtung für das Einpressen von Schmiermittel in einen Ringspalt zwischen dem Schaft und dem inneren Stülpschlauchabschnitt auszubilden, wobei zumindest eine im wesentlichen radial verlaufende Bohrung oder Perforation in der Wandung des Schafts vorgesehen ist, die sich in den Ringspalt öffnet und an einer Schmiermittelfördervorrichtung angeschlossen ist. Auf diese Weise wird bei geringem baulichen Aufwand Schmiermittel unmittelbar in den Ringspalt gefördert.

Weitere vorteilhafte Ausgestaltungen sind Gegenstand der übrigen Unteransprüche.

Die Erfindung wird nachstehend anhand eines bevorzugten Ausführungsbeispiels unter Bezugnahme auf die begleitenden Zeichnungen näher erläutert. Es zeigen:
Fig. 1 ein Endoskop mit integrierter Schaftschmierung und Reibradantrieb gemäß einem beverzugten Ausführungsbeispiel der Erfindung,

Fig. 1a eine Vergrößerung der Verbindungsstelle zwischen einem Auslaßschlauch eines Schmiermittel-Druckbehälters und einem Zuführschlauch des Endoskops,

Fig. 2 ein Endoskop gemäß dem bevorzugten Ausführungsbeispiel der Erfindung mit einer weiteren alternativen Antriebseinrichtung.

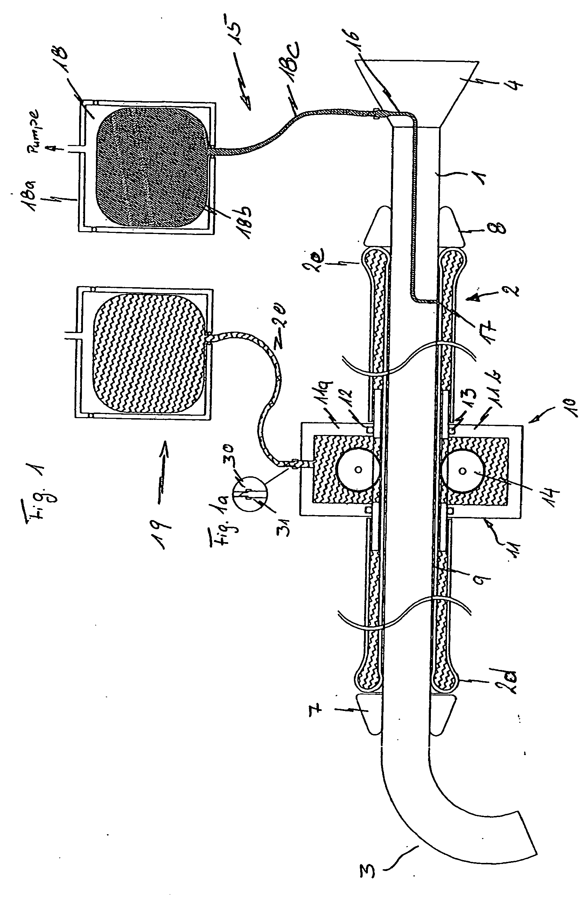

In Fig. 1 ist der schematische Seitenriß eines Endoskops gemäß einem Ausführungsbeispiel der Erfindung dargestellt.

Demzufolge besteht das erfindungsgemäße Endoskop aus einem Endoskopschaft 1, der über eine Länge von 1 bis 2 m (in der Regel ca. 1600 mm) mit einem Stülpschlauch 2 der Doppelstülpbauart bzw. mit beidseitiger Umstülpung ummantelt ist. Das distale Ende 3 des Endoskopschafts 1 besteht aus einem in alle Richtungen abwinkelbaren oder krümmbaren Endstück an dessen Spitze eine Beleuchtungseinrichtung sowie ein Kamerachip angeordnet sind (beides nicht gezeigt), der über elektrische Leitungen, die innerhalb des Endoskopschafts 1 verlegt sind, an einer Kamerasteuerung am hinteren Endabschnitt des Endoskops verbunden ist. Die Kamera ist wiederum über einen Videoprozessor an einen Monitor angeschlossen.

Alternativ hierzu kann natürlich auch eine Optik an der Spitze des abwinkelbaren Endstücks 3 vorgesehen sein, das über Lichtleiter z.B. Glasfaserbündel innerhalb des Endoskopschafts 1 mit der Kamerasteuerung verbunden ist.

Im Endoskopschaft 1 erstreckt sich ferner zumindest zwei Kanäle oder Schläuche, die sich an der vordersten Spitze des krümmbaren Endstücks 3 nach außen öffnen und die am hinteren Endabschnitt des Endoskops einen Einführanschluß bzw. manuell steuerbare Ventile besitzen. Einer dieser Kanal dient der Luft-oder Gaszufuhr und als Zuführkanal von Reinigungsfluid für das Spülen zu untersuchender Stellen des Hohlraums, der andere als Arbeitskanal zum Einführen von medizinischen Arbeitsgeräten, die bis zum distalen Ende vorgeschoben werden können, um beispielsweise Gewebeproben zu entnehmen oder chirurgische Eingriffe vorzunehmen.

Zur Betätigung des Endstücks 3 ist dieses über innerhalb des Endoskopschafts 1 verlegte Bowdenzüge (nicht gezeigt) mit einer manuellen Betätigungseinrichtung 4 wirkverbunden, die an einem Endabschnitt des Endoskopschafts 1 innerhalb eines Gehäuses untergebracht ist.

Wie insbesondere aus Fig. 1 zu entnehmen ist, besteht der erfindungsgemäße Stülpschlauch 2 aus einem inneren Schlauchabschnitt 2a, der durch eine Antriebs- und Führungshülse bzw. ein Schlauchführungsteil 5 gleitfähig hindurchgeführt und in seinem vorderen Bereich (Umstülpbereich) zu einem vorderen äußeren Schlauchabschnitt 2b umgestülpt ist. Der vordere äußere Schlauchabschnitt 2b ist dabei zu der Antriebs- und Führungshülse (Schlauchführungsteil) 5, welche aus einem steifen Material, vorzugsweise einem Kunststoffmaterial besteht, zurückgeführt und an einem axialen Ende an der Antriebshülse 5 derart befestigt, daß diese zwischen dem inneren 2a und äußeren Schlauchabschnitt 2b zu liegen kommt.

In einem hinteren Bereich (Umstülpbereich) ist der innere Schlauchabschnitt 2a zu einem hinteren äußeren Schlauchabschnitt 2c umgestülpt, der zu der Antriebshülse 5 zurückgeführt und an einem axialen Ende der Antriebshülse 5 fixiert ist. Die Antriebshülse 5 dient zum einen als Führungselement für den inneren Schlauchabschnitt 2a, um Aufwerfungen und Falten- bzw. Schuppenbildung zu verhindern und zum anderen als Verbindungsstück des vorderen äußeren 2b und hinteren äußeren Schlauchabschnitts 2c, wobei ein Mittenbereich der Antriebshülse 5 exponiert, d.h. nicht von dem Stülpschlauch 2 überdeckt verbleibt. In diesem Mittenabschnitt weist die Antriebshülse 5 zumindest eine Öffnung, vorzugsweise einen in Axialrichtung sich erstreckenden Längsschlitz 6 vorbestimmter Breite auf. Vorliegend sind vier, im gleichen Winkelabstand zueinander angeordneter Längsschlitze 6 vorgesehen. Des weiteren hat die Antriebshülse 5 an ihrer Innenseite eine Anzahl von durchgehenden Längsnuten 5a, die sich an den Stirnseiten der Antriebshülse 5 öffnen. Diese Längsnuten 5a können dabei entweder achsparallel oder spiralförmig gezogen sein.

Wie insbesondere aus der Fig. 1 zu erkennen ist, ist das Material, d.h. die Materialart und die Materialstärke des Stülpschlauchs 2 derart gewählt, daß sich im vorderen und hinteren Stülpbereich 2d, 2e jeweils eine wulstförmige Aufweitung infolge einer Materialanhäufung an der Umstülpung ausbildet, die im inneren Bereich zu einer vorbestimmten Einengung des Innendurchmessers des Stülpschlauchs 2 führt.

Gemäß der Fig. 1 liegt der Stülpschlauch 2 an seinem vorderen Stülpbereich 2d gleitend an einem kegelförmigen vorderen Klemmstück 7 an, das zumindest in Axialrichtung fest auf dem Endoskopschaft 1 sitzt und sich in Eindringrichtung des Endoskops konisch verjüngt. Der hintere Stülpbereich 2e des Stülpschlauchs 2 liegt ebenfalls an einem hinteren Klemmstück 8 gleitend an, das in diesem besonderen Ausführungsbeispiel genau wie das vordere Klemmstück 7 fest auf dem Endoskopschaft 1 sitzt und auch eine entsprechende Form besitzt. Das hintere Klemmstück 8 verjüngt sich jedoch konisch entgegengesetzt zur Eindringrichtung des Endoskops. An dieser Stelle sei jedoch darauf hingewiesen, daß zumindest das hintere Klemmstück 8 eine beliebige äußere Form aufweisen kann, da es nicht in den zu untersuchenden Hohlraum eingeführt wird.

Aus der Fig. 1 ist ferner deutlich zu entnehmen, daß sich zwischen dem inneren Schlauchabschnitt 2a des Stülpschlauchs 2 und dem Endoskopschaft 1 ein schmaler Ringspalt 9 ausbildet, der axial durch die beiden Wülste in den Umstülpbereichen 2d, 2e des Stülpschlauchs 2 begrenzt wird, die sich dichtend an die äußere Mantelfläche des Endoskopschafts 1 anlegen.

Um die Antriebshülse 5 ist in deren Mittenbereich eine Antriebs- oder Vorschubeinrichtung 10 angeordnet. Diese Vorschubeinrichtung 10 besteht vorliegend aus einem Gehäuse 11 vorzugsweise aus einem Kunststoff oder einer nichtrostenden Metallegierung, in welchem der nachfolgend noch beschriebene Antriebsmechanismus für den Stülpschlauch 2 gemäß dem ersten zweiten und dritten Ausführungsbeispiel der Erfindung untergebracht ist. Das Gehäuse 11 selbst besteht aus zwei schalenförmigen Gehäusehälften 11a, 11b, die an einem freien Kantenabschnitt längs der Antriebshülse 5 aufklappbar mittels eines nicht gezeigten Gelenks oder Scharniers aneinanderscharniert und an dem gegenüberliegenden freien Kantenabschnitt mittels einer ebenfalls nicht gezeigten Riegeleinrichtung verriegelbar sind. Alternativ hierzu können die Gehäusehälften 11a, 11b natürlich auch vollständig geteilt und mittels zweier Riegeleinrichtungen verbindbar sein. An den bezüglich der Antriebshülse 5 querlaufenden Seitenwandungen des Gehäuses 11 befinden sich an jeder Gehäusehälfte 11a, 11b der äußeren Querschnittsform der Antriebshülse 5 angepaßte Aussparungen, die in zusammengeklapptem und verriegeltem Zustand des Gehäuses 11 jeweils ein in sich geschlossenes Ausnehmungs- oder Öffnungsprofil entsprechend der Außenkontur der Antriebshülse 5 bilden und diese nach Außen dichtend umschließen. Die Aussparungen sowie die freien Kanten der Gehäusehälften 11a, 11b sind an ihren jeweiligen Schnitt- bzw. Kantenflächen mit Nuten 12 (nicht weiter gezeigt) versehen, in die Dichtungsstreifen 13 eingeklemmt oder geklebt sind, die sich bei Zusammenklappen der Gehäusehälften 11a, 11b dichtend an die Mantelfläche der Antriebshülse 5 anlegen, um so einen Gehäuseinnenraum dichtend abzuschließen.

Vorliegend besitzt die Antriebs- und Schlauchführungshülse 5 im Querschnitt eine kreisförmige Außenkontur entsprechend dem Kreisquerschnitt des Stulpschlauchs 2, um eine möglichst dichte und spannungsfreie Verbindung mit dem Stülpschlauch zu gewährleisten. Sie kann aber auch eine linsenförmige oder anderweitige Querschnittsform in Abhängigkeit der Konstruktion des Antriebsmechanismus aufweisen.

Dieser Antriebsmechanismus umfaßt gemäß dem bevorzugten Ausführungsbeispiel der Erfindung einen nicht weiter gezeigten Antriebsmotor, der über einen Getriebezug (ebenfalls nicht dargestellt) auf eine Anzahl von Reibrädern 14 einwirkt. Diese Reibräder 14 sind an den beiden Gehäusehälften 11a, 11b jeweils derart gelagert, daß sie in zusammengeklapptem bzw. geschlossenem Zustand des Gehäuses 11 jeweils in einen Längsschlitz 6 der Antriebshülse 5 eindringen und mit einer vorbestimmten vorzugsweise über Federn einstellbaren Anpreßkraft auf dem inneren Schlauchabschnitt 2a des Stülpschlauchs 2 reibschlüssig anliegen. Vorzugsweise ist die Lauffläche jedes Antriebsrads 14 mit einem Haftbelag (Beschichtung mit Keramikteilchen oder Metallgranulat vorbestimmter Körnung) zur Erhöhung des Reibungskoeffizienten zwischen dem Stülpschlauch 2 und dem Antriebsrad 14 überzogen. Alternativ können die Reibräder 14 natürlich auch als Ganzes aus einem entsprechenden Material, beispielsweise Schleifkeramik, gefertigt sein. Die Anpreßkraft ist ferner in Abhängigkeit von dem Material und der Wandstärke des Stülpschlauchs 2 so gewählt, daß ein gleichmäßiger, im wesentlichen schlupffreier Vortrieb des Stülpschlauchs 2 über die Reibräder 14 gewährleistet ist.

Das Endoskop gemäß dem bevorzugten Ausführungsbeispiel der Erfindung ist des weiteren mit einem Schmiersystem zur Schmierung der Relativ-Gleitbewegung zwischen dem Endoskopschaft 1 und dem inneren Schlauchabschnitt 2a, sowie zwischen dem inneren und äußeren Schlauchabschnitt 2a, 2b, 2c ausgerüstet, das nachfolgend beschrieben wird.

Dieses Schmiersystem umfaßt eine erste Schmiermittelförder- und zuführvorrichtung 15 mit einer Druckluftpumpe (nicht gezeigt) bzw. einem Druckluftanschluß, der an einen Druckbehälter 18 angeschlossen ist, um diesen mit Druckluft zu beaufschlagen bzw. zuzuführen. Dieser Druckbehälter 18 besitzt einen aufklapp- oder entfernbaren Deckel 18a, mittels dem eine Be-und Entladeöffnung verschließbar ist. Innerhalb des Druckbehälters 18 befindet sich ein entnehmbarer bzw. auswechselbarer Kunststoff- oder Gummibeutel 18b oder alternativ ein Schmiermittelfaltenbalg, der mit dem zu verwendenden Schmiermittel gefüllt ist. Dieser Beutel oder Balg 18b hat einen Auslaßschlauch 18c, der durch eine, vorzugsweise der Anschlußstelle der Pumpe gegenüberliegende Bohrung des Druckbehälters 18 geführt ist. Die Bohrung schließt dabei dichtend am Auslaßschlauch 18c ab, wofür in diesem Ausführungsbeispiel ein Dichtungskeder an der Bohrung angebracht ist. Zusätzlich oder alternativ zu dieser Dichtung legt sich der Beutel 18 dichtend an der Druckbehälterwand an, so daß die Auslaßbohrung im wesentlichen luftdicht von der Anschlußstelle der Pumpe, welche sich vorzugsweise im Deckel 18a befindet, durch den Beutel 18b abgetrennt wird.

Am freien Ende des Auslaßschlauchs 18c ist ein Kupplungsstück 30 vorgesehen, das mit einem entsprechenden Kupplungsgegenstück 31 eines Zuführschlauchs 16 des Endoskops verbindbar ist, das aus dem hinteren Endabschnitt des Endoskopschafts 1 insbesondere aus dem Gehäuse der Betätigungseinrichtung 4 seitlich herausragt. Der Zuführschlauch oder Kanal 16 ist hierbei, wie in der Fig. 1 andeutungsweise gezeigt wird, innerhalb des Endoskopschafts 1 bis zu einer im Endoskopschaft 1 ausgebildeten Auslaßbohrung 17 im Bereich des Stülpschlauchs 2 weiterverlegt.

Die beiden Kupplungsstücke des Auslaß- 18c und des Zuführschlauchs 16 sind in der Fig. 1a schematisch dargestellt.

Demzufolge besitzt das eine Kupplungsstück 30 einen Kupplungsflansch mit einer Überwurfmutter oder einem Bajonettverschluß, wobei die Mündung des Auslaßschlauchs 18c durch eine Membran verschlossen ist. Das andere Kupplungsgegenstück 31 hat ein der Überwurfmutter oder dem Bajonettverschluß entsprechendes Gegenelement sowie eine nadelförmig oder mit einer Schneide ausgebildeten Kanüle als äußerstes Ende des Zuführschlauchs 16. Beim Zusammenstecken der beiden Kupplungsstücke 30, 31 durchdringt die Kanüle die Membran und stellt so eine Fluidverbindung zwischen dem Auslaßschlauch 18c und dem Zuführschlauch 16 her.

Es sei darauf hingewiesen, daß die Anordnung der Kupplung 30, 31 keinesfalls am Endoskop sein muß. Das eine Kupplungsstück 30 kann beispielsweise auch in der Bohrung des Druckbehälter 18 integriert sein, wobei in diesem Fall das Gegenstück 31 unmittelbar am Beutel 18b plaziert werden kann, so daß bei Einsetzten des Beutels 18b automatisch eine Verbindung zum Endoskop hergestellt wird. In dessen ist die erste Ausführungsvariante zu bevorzugen, da hierdurch bereits bei Zusammenstecken der beiden Schläuche 18c und 16 der Auslaßschlauch 18c über seine gesamte Länge mit Schmiermittel vorab gefüllt ist und damit eine nachträgliche Entlüftung des Schmiersystems nicht mehr erforderlich ist.

Wie ferner in der Fig. 1 gezeigt ist, durchdringt der Zuführschlauch 16 die Auslaßbohrung 17, derart, daß kein Schmiermittel in das Innere des Endoskopschafts 1 auslecken kann und mündet dabei in den Ringspalt 9 zwischen dem inneren Stülpschlauchabschnitt 2a und dem Endoskopschaft 1, der durch den vorderen und hinteren Stülpbereich 2d, 2e axial fluiddicht begrenzt wird.

Alternativ zu der vorstehend beschriebenen Ausgestaltung des Schmiersystems ist es natürlich auch möglich, daß der Zuführschlauch 16 bzw. der Auslaßschlauch 18c direkt an einen Anschluß am Endoskopschaft 1 angeschlossen ist, so daß sich das zugeförderte Schmiermittel innerhalb des gesamten Endoskopschafts 1 ausbreiten kann. D.h., daß der Endoskopschaft 1 in diesem Fall selbst als Teil des Zuführkanals 16 dient, wobei das Schmiermittel über den Zuführschlauch 16 und den Endoskopschaft 1 zur Auslaßbohrung 17 gefördert wird. Dabei müssen dann sämtliche "Innereien" des Endoskopschafts 1 mit einer spezielen Beschichtung oder Isolierung überzogen oder in Kanälen geführt sein, um eine Kontamination und damit verbundene Beschädigung durch das relativ aggressive Schmiermittel zu verhindern. Anstelle der zumindest einen Auslaßbohrung 17 kann dabei natürlich auch eine Perforation des Endoskopschafts 1 an der entsprechenden Stelle vorgesehen sein, wobei im Endoskopschaft 1 eine eigene Schmiermittelleitung 16 mit meheren Auslässem zur Schaftoberfläche verlegt sein kann.

Der Förderdruck der ersten Schmiermittelfördervorrichtung 15 ist so gewählt, daß der entstehende Staudruck innerhalb des Ringspalts 9 zwischen dem Endoskopschaft 1 und dem inneren Schlauchabschnitt 2a, kleiner ist als der Anpreßdruck der wulstförmigen Umstülpbereiche 2d, 2e des Stülpschlauchs 2 auf den Endoskopschaft 1. In diesem Fall wirken die Umstülpbereiche 2d, 2e zusammen mit dem Schaft 1 unmittelbar als Dichtungen, die eine Leckage an Schmiermittel verhindern. Des weiteren hat dies den Vorteil, daß diese Art von Dichtung vorerst nicht entlang des Endoskopschafts 1 oder mit entsprechendem Anpreßdruck am vorderen Endstück 7 gleiten muß sondern systementsprechend am Endoskopschaft 1 abwälzt, wodurch eine Erhöhung der notwendigen Vorschubkraft auf den inneren Schlauchabschnitt 2a zur Überwindung von Reibkräften unterbleibt. Dies wiederum führt zu einer verringerten notwendigen Anpreßkraft der Reibräder 14 auf den inneren Schlauchabschnitt 2a. Auf diese Weise wird auch gewährleistet, daß der Endoskopschaft 1 innerhalb des Stülpschlauchs 2 drehbar bleibt, um so bei anormalen Darmwindungen ein Durchfahren durch den Darm möglich zu machen.

Des weiteren umfaßt das Schmiersystem eine zweite Schmiermittelförder- und Zuführvorrichtung 19, die über einen Zuführschlauch oder Kanal 20 mit dem Gehäuse 11 der Antriebseinrichtung 10 verbunden ist. Der konstruktive Aufbau der zweiten Schmiermittelförder- und Zuführvorrichtung 19 entspricht im Prinzip dem der ersten Schmiermittelförder- und Zuführvorrichtung 15 bzw. den hierzu beschriebenen Alternativen, so daß an dieser Stelle auf die entsprechenden Textstellen verwiesen werden kann.

Das zweite Schmiermittelförder- und Zuführeinrichtung 19 ist hingegen wie bereits angedeutet, an das Antriebsgehäuse 11 angeschlossen, um das geförderte Schmiermittel in den dicht verschlossenen Innenraum des Antriebsgehäuses 11 zu pressen, wobei die Kupplung unmittelbar an dem Gehäuse 11 angeordnet ist. Dieses Schmiermittel hat ferner über die Öffnungen oder Längsschlitze 6 in der Antriebshülse 5 Zugang in den Hohlraum zwischen dem inneren und äußeren Schlauchabschnitt 2a, 2b, 2c des Stülpschlauchs 2, um somit die Relativ-Gleitbewegung zwischen dem inneren und äußeren Schlauchabschnitt 2a, 2b, 2c zu schmieren. Darüber hinaus bewirkt das Schmiermittel innerhalb des Antriebsgehäuses 11 natürlich auch eine Schmierung des Antriebsmechanismus, d.h. der Lagerung der Reibräder 14 selbst.

Zur Funktions- und Betriebsweise des Endoskops gemäß dem bevorzugten Ausführungsbeispiel der Erfindung wird folgendes ausgeführt:

Zum Einführen des Endoskops, bzw. des Endoskopschafts 1 in den Darm eines Patienten über dessen Anus wird der Antriebsmechanismus, d.h. das Antriebsgehäuse 11 auf einen Sockel montiert, der bezüglich einer Platte, auf der der Patient zumindest teilweise liegt, höhenverstellbar und in jede Richtung schwenkbar ist, so daß das distale Ende 3 des Endoskopschafts 1 entsprechend dem individuellen Körperbau des Patienten angepaßt werden kann. Anschließend wird das distale Ende 3 des Endoskopschafts 1, d.h. ein kurzes Stück in den Anus eingeschoben, solange, bis der Stülpschlauch 2 den Schließmuskel passiert hat. Hierauf wird über den Endoskopschaft 1 bzw. ein darin geführter Kanal CO2-Gas in den Darm eingeleitet, um diesen zu entfalten, worauf die Antriebseinrichtung 10 betätigt wird, um die Reibräder 14 mit einer vorbestimmbaren oder während des Betriebs auch änderbaren Umdrehungszahl anzutreiben. Entsprechend dieser Umdrehungszahl wird der innere Schlauchabschnitt 2a langsam in Eindringrichtung des Endoskops vorgeschoben, wobei er sich im vorderen Umstülpbereich 2d kontinuierlich zu dem vorderen, äußeren Schlauchabschnitt 2b umstülpt und dabei die Darmwandung seitlich auskleiden kann. Gleichzeitig wird die Antriebskraft der Reibräder 14 auch auf den Endoskopschaft 1 übertragen, da durch die Anpreßkraft der Reibräder 14 auf den relativ weichen inneren Stülpschlauchabschnitt 2a dieser auf den Endoskopschaft 1 angedrückt wird und diesen trotz des dazwischen sich ausbreitenden Schmiermittels durch Reibungskräfte mitnimmt. D. h., durch den Vorschub des inneren Schlauchabschnitts 2a wird der Endoskopschaft 1 durch Reibungskräfte zwischen dem Stülpschlauch 2 und dem Schaft 1, gegebenenfalls auch geringfügig durch Druckkräfte in Vorschubrichtung zwischen dem vorderen Umstülpbereich 2d und dem vorderen Klemmstück 7 des Endoskopschafts 1 mitgeschoben und somit in den Darm eingeführt.

Um nunmehr die Vorschubgeschwindigkeit des Endoskopschafts 1 nicht über die Eindringgeschwindigkeit des Stülpschlauchs 2 anwachsen zu lassen, wie bereits in der Beschreibungseinleitung ausführlich erläutert wurde, ist ein Rückhalten oder Abbremsen des Endoskopschafts 2 gegenüber dem inneren Schlauchabschnitt 2a erforderlich. Dies geschieht vorliegend über das hintere Klemmstück 8, an welches sich der hintere Umstülpbereich 2e des Stülpschlauchs 2 anlegt, der sich zwangsläufig mit der gleichen Geschwindigkeit bewegt, wie der vordere Umstülpbereich 2d und der somit die Vorschubgeschwindigkeit des Endoskopschafts 1 bezüglich der Eindringgeschwindigkeit des Stülpschlauchs 2 synchronisiert (d.h. das hintere Klemmstück 8, das fest auf dem Endoskopschafts 1 sitzt, hält diesen zurück und leitet dabei die Reaktionskraft in den hinteren Stülpschlauchabschnitt 2c ein). Die Eindringgeschwindigkeit des Stülpschlauchs 2 ist demzufolge gleich groß wie die des Endoskopschafts 1.

An dieser Stelle sei darauf hingewiesen, daß während des Synchronisationsvorgangs der Vorschubbewegungen des Stülpschlauchs 2 und des Endoskopschafts 1 dieser an den vorderen und hinteren Wülsten wie auch im Bereich der Antriebshülse 5 an dem inneren Stülpschlauchabschnitt 2a gleitet, wobei jedoch die Gleitschwindigkeit des Endoskopschafts 1 bezüglich der vorderen und hinteren Wülste, d.h. an den Umstülpbereichen 2d und 2e bzw. gegenüber dem inneren Schlauchabschnitt 2a lediglich der Relativgeschwindigkeit beider Bauteil entspricht, die nur halb so groß ist, wie die Absolutgeschwindigkeit des inneren Stülpschlauchsabschnitts 2a.

Um hierbei die Bremskräfte auf den hinteren Umstülpbereich 2e nicht zu groß werden zu lassen und damit ggf. ein Zusammenstauchen des Stülpschlauchs 2 im hinteren äußeren Schlauchabschnitt 2c durch die aufzubringenden Bremskräfte zu verhindern, ist es erforderlich, die Reibungskräfte zwischen dem Endoskopschaft 1 und dem inneren Schlauchabschnitt 2a möglichst gering zu halten.

Wie vorstehend bereits beschrieben wurde, bildet sich in dem Ringspalt 9 zwischen dem Schaft 1 und dem inneren Schlauchabschnitt 2a auch im Bereich der Antriebseinrichtung 10 ein nahezu durchgehender Schmierfilm aus, der gerade noch einen Vortrieb des Endoskopschafts 1 im wesentlichen durch die Reibräder 14 ermöglicht, jedoch auch die entstehende Reibung bei der besagten Relativbewegung zwischen den beiden Bauteilen verringert. Darüber hinaus müssen die Umstülpbereiche 2d, 2e und die vorderen und hinteren Klemmstücke 7, 8 aufgrund des geringen Vortriebsanteils auf das vordere Klemmstück 7 bzw. der geringen notwendigen Bremskraft auf das hintere Klemmstück 8 nicht so stark aufeinandergepreßt werden, um beispielsweise eine Dichtwirkung zu erzielen, wie dies bisher im Stand der Technik der Fall war, da dieser Dichtungseffekt bereits durch das Zusammemwirken zwischen den wulstförmigen Umstülpbereichen 2d, 2e und dem Endoskopschaft 2 erreicht wird. Im übrigen wird durch die zweite Schmiermittelfördervorrichtung 19 das Schmiermittel in den Hohlraum zwischen dem inneren und äußeren Schlauchabschnitt 2a, 2b, 2c mit einem vorbestimmten Druck gepreßt, so daß hierdurch die Reibungskräfte weiter verringert werden. Hierbei reicht unter Umständen aufgrund geringer Leckage ein anfänglicher Schmiervorgang zur Befüllung des Hohlraums zwischen dem äußeren und inneren Stülpschlauchabschnitt aus, d.h. daß während zumindest einer Behandlungsdauer nicht unbedingt Schmiermittel nachgepreßt werden muß.

Das Zusammenwirken der Reibräder 14 als ein gemeinsamer kontinuierlicher Antrieb für den Stülpschlauch 2 und für den Endoskopschaft 1, der vorderen und hinteren Wülste als Dichtungen zusammen mit dem Endoskopschaft 1 sowie des durchgehenden Schmierfilms zwischen dem inneren Schlauchabschnitt 2a und dem Schaft 1 erlaubt somit einen äußerst exakten und präzise steuerbaren Vortrieb des Endoskopschafts 1, der dessen Handhabung im Vergleich zum Stand der Technik wesentlich erleichtert.

An dieser Stelle sei darauf hingewiesen, daß an Stelle des hinteren Klemmstücks 8 auch eine zusätzliche externe Synchronisations-Antriebseinrichtung vorgesehen werden kann, die über eine Anzahl von Reibrädern auf den Endoskopschaft 1 an dessen hinterem Endabschnitt einwirkt und somit die kontinuierliche Eindringbewegung des Endoskops gewährleistet.

Des weiteren kann als externer Synchronisations-Antrieb an Stelle der Reibradkonstruktion ein Spindelantrieb für den geregelten kontinuierlichen Vortrieb des Endoskopschafts 1 vorgesehen sein.

Das Zurückziehen des Endoskops erfolgt im wesentlichen auf die gleiche Weise wie der Eindringvorgang, wobei in diesem Fall jedoch das vordere Klemmstück 7 die Synchronisation der Bewegungsgeschwindigkeiten zwischen dem Endoskopschaft 1 und dem Stülpschlauch 2 übernimmt, während das hintere Klemmstück 8 weitestgehend belastungsfrei ist.

Abschließend sei bezüglich des bevorzugten Ausführungsbeispiels der Erfindung darauf hingewiesen, daß insbesondere der Antrieb des Endoskops durch Bauteile unterschiedlich zu den Reibrädern 14 erfolgen kann. So könnten beispielsweise für die Vorschubeinrichtung 10 des Stülpschlauchs 2 Zahnräder vorgesehen sein, die auf den inneren Schlauchabschnitt 2a einwirken. All diese Varianten haben jedoch gemeinsam, daß die in Radialrichtung wirkende Anpreßkraft ausreicht, um den inneren Schlauchabschnitt 2a gegen den Endoskopschaft 1 für dessen kontinuierlichen Antrieb trotz des Vorhandenseins von Schmiermittel zu drücken, wodurch die exakte Positionierbarkeit des distalen Endes an einer zu untersuchenden Stelle ermöglicht wird. Hierzu wird auf die nachfolgend beschriebenen alternativen Antriebseinrichtungen verwiesen .

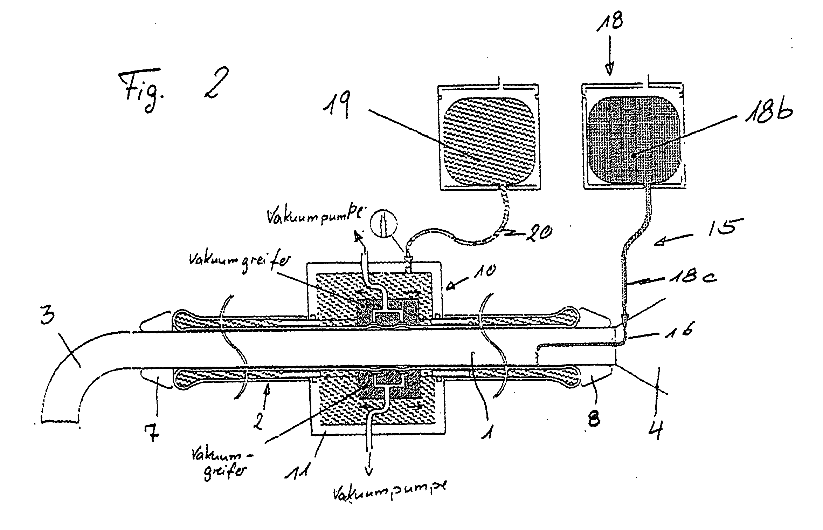

Gemäß der Fig. 2 besteht die erste alternative Antriebseinrichtung aus einem Antriebsgehäuse 11, das um die Schlauchführungshülse 5 wie im erst und zweiten Ausführungsbeispiel gelegt ist und in dem zumindest zwei Vakuumgreifer in Axialrichtung des Endoskopschafts 1 alternierend bewegbar angeordnet sind. Die Vakuumgreifer bestehen aus kleinen, durch die Öffnungen in der Antriebshülse 5 durchgreifenden, an dem inneren Stülpschlauchabschnitt 2a dicht anliegenden Klötzchen mit Saugnäpfen, die auf der mit dem Schlauch 2a in Kontakt stehenden Seite der Klötzchen ausgebildet sind und die durch einen von einer Vakuumpumpe erzeugten Unterdruck das Schlauchmaterial im Bereich des inneren Stülpschlauchabschnitts 2a ansaugen und so eine kraft-und formschlüssige Verbindung zum inneren Schlauchabschnitt 2a herstellen. Die Vakuumpumpe (nicht gezeigt) ist hierfür an einen Vakuumanschluß angeschlossen, der im Gehäuse 11 der Antriebseinrichtung 10 ausgebildet und über Kanäle innerhalb der Klötzchen mit den Suagnäpfen verbunden ist. Die Bewegung der Klötzchen, welche durch eine nicht näher gezeigte Bewegungsmechanik bewerkstelligt wird, sowie das Erzeugen des Unterdrucks innerhalb der Saugnäpfe wird derart gesteuert, daß die Klötzchen abwechselnd bzw. bei mehr als zwei Klötzchen in gleichmäßigen Abständen fortlaufend mit einsprechend synchronem Ansaugen und Freigeben des inneren Stülpschlauchabschnitts 2a hin- und herbewegt werden und dabei eine quasi kontinuierliche und gleichmäßige Vorschubbewegung des Stülpschlauchs 2 erzeugen.

Wie in der Fig. 2 gut zu erkennen ist, bewirkt das Vakuumisieren der Saugnäpfe ein geringfügiges Abheben des inneren Stülpschlauchabschnitts 2a vom Endoskopschaft. Die Vorschubkraft auf den Endoskopschaft 1 erfolgt in diesem Ausführungsbeispiel im wesentlichen wie bei den bisherigen Ausführungen. Die gesamte Anordnung ist dabei wie im vorstehen beschrieben Ausführungsbeispiel mit einem Schmiermittel gefüllt, das die Schmierung des Stülpschlauchs 2 übernimmt.

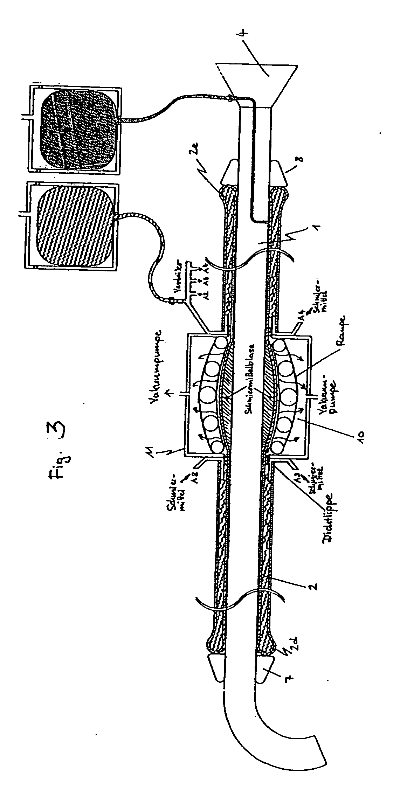

Die Fig. 3 zeigt eine weitere alternative Ausführungsvariante, bei der durch Erzeugen eines Unterdrucks eine Antriebskraft auf den Stülpschlauch anlegbar ist.

Wie aus der Fig. 3 zu entnehmen ist, bildet der beidseitig umgestülpte Stülpschlauch 2 zusammen mit einem Gehäuse 11 des Antriebsmechanismus ein einstückiges Bauteil ohne die Anordnung einer Antriebshülse. D.h., daß in diesem alternativen Ausführungsbeispiel die freien Enden der äußeren vorderen und hinteren Schlauchabschnitte 2b und 2c direkt an den Seitenwänden des Antriebsgehäuses 11 dichtend befestigt sind.

Der Antriebsmechanismus wird dabei durch eine Art Raupenantrieb bestehend aus zumindest einem elastischen Endlosband gebildet, welches über zumindest zwei in Vorschubrichtung voneinander beabstandeter Räder gespannt und angetrieben wird. Die gesamte Antriebseinrichtung ist in dem vorzugsweise in Längsrichtung zweigeteilten, luftdicht verschließbaren Gehäuse untergebracht, welches einen Sauganschluß aufweist. Das Endlosband weist an seiner Oberfläche eine Anzahl von Öffnungen oder eine Perforation auf. Der Sauganschluß ist über eine Schlauchleitung an eine nicht gezeigte Vakuumpumpe angeschlossen.

Das Gehäuse 11 selbst ist auf seiner dem inneren Schlauchabschnitt 2a zugewandten Seite offen, wobei das Endlosband derart geführt ist, daß es die offene Seite des Gehäuses 11 möglichst dicht verschließt. Spalte zwischen den Seitenrändern des Endlosbands und dem Gehäuse 11 können durch geeignete Dichtlippen verschlossen sein, die an den Gehäusewänden befestigt sind und an denen das Endlosband gleitet. Des weiteren ist das Gehäuse 11 bezüglich des daran befestigten Stülpschlauchs 2 derart angeordnet, daß sich die nach außen exponierte Seite des Endlosbands mit einem geringen Spalt oder Abstand oberhalb des inneren Schlauchabschnitts 2a ausrichtet.

Im Betrieb dieser Vorrichtung wird der Innenraum des Gehäuses 11 durch die Vakuumpumpe evakuiert, wobei Luft durch die Perforation in dem Endlosband angesaugt wird. Hierbei wird gleichzeitig der innere Schlauchabschnitt 2a mit angesaugt, der sich dichtend an die Außenseite des Endlosbands anlegt. Durch dieses Abdichten der Perforation erhöht sich der Unterdruck innerhalb des Gehäuses derart, daß sich das Endlosband aufgrund dessen Elastizität nach innen wölbt und dabei den inneren Schlauchabschnitt 2a mitnimmt, wie in der Fig. 3 deutlich gezeigt wird.

Durch diese Maßnahme wird der innere Schlauchabschnitt 2a über die Länge des Endlosbands fest an diesem gehalten und gleichzeitig vom Endoskopschaft 1 beabstandet. Der Vortrieb des Endoskopschafts 1 erfolgt somit im wesentlichen durch die Anlagekräfte, die bei einer Vorschubbewegung des vorderen äußeren Schlauchabschnitts 2b durch das Endlosband am vorderen Umstülpbereich 2d auf das vordere Klemmstück 7 aufgebracht werden, wobei der innere Schlauchabschnitt 2a insbesondere im Bereich des Antriebseinheit kontaktlos bleibt. Aufgrund der geringen Reibungskräfte ist die notwendige Vorschubkraft auf das vordere Klemmstück 7 relativ niedrig, so daß mit keinem Schuppen (Faltenbildung) im vorderen Abschnitt des Stülpschlauchs 2 zu rechnen ist. Im übrigen wird wie beim ersten und zweiten Ausführungsbeispiel ein Schmiermittel in den Ringspalt 9 gepreßt, wodurch die Reibung insbesondere im Bereich der vorderen und hinteren Dichtungswülste 2d, 2e weiter verringert wird.

Beide alternative Antriebsvarianten sefen demzufolge einen kontinuierlich bzw. quasikontinuierlich arbeitenden mechnisch auf den Stülpschlauch 2 einwirkenden Antriebsmechanismus vor, der eine exakt steuerbare Fortbewegung des Endoskops gewährleistet.

## Patentansprüche

1. Endoskop mit einem Stülpschlauchsystem und einem inneren Schaft (1) oder einem schaftähnlichen Bauteil, der in einem an beiden axialen Endbereichen jeweils gestülpten Schlauch (2) gleitend geführt ist, der durch eine Antriebseinrichtung (10) fortbewegbar ist, die auf einen inneren Schlauchabschnitt (2a) des Stülpschlauchs (2) einwirkt, wobei eine Schmiervorrichtung (15,16,17,18) für das Einpressen von Schmiermitteln in einen Ringspalt (9) zwischen dem Schaft (1) und dem inneren Stülpschlauchabschnitt (2a) vorgesehen ist, **dadurch gekennzeichnet, dass** dafür zumindest eine im wesentlichen radial verlaufende Bohrung (17) oder Perforation in der Wandung des Schafts (1) vorgesehen ist, die sich in den Ringspalt (9) öffnet und die an einer Schmiermittelfördervorrichtung (15) angeschlossen ist.

2. Endoskop nach Anspruch 1, **dadurch gekennzeichnet, dass** die Zufuhr an Schmiermittel zu der Bohrung (17) oder Perforation über den Hohlraum innerhalb des Schafts (1) erfolgt, der somit einen Abschnitt einer Schmiermittelzuführleitung (16) ausbildet.

3. Endoskop nach Anspruch 1, **dadurch gekennzeichnet, dass** innerhalb des Schafts (1) eine Zuführleitung (16) verlegt ist, die an ihrem einen Ende an die Bohrung (17) oder Perforation und an ihrem anderem Ende an eine externe Schmierleitung (18c) angeschlossen ist, die in einen Schmiermittel druckbehälter (18) mündet.

4. Endoskop nach einem der vorhergehenden Ansprüche **dadurch gekennzeichnet, dass** der Stülpschlauch (2) einen vorderen und hinteren Stülpbereich (2d, 2e) hat, die jeweils die Form einer den Innendurchmesser des Stülpschlauchs (2) verengenden Aufwülstung annehmen, derart, dass sich die Stülpbereiche (2d, 2e) dichtend gegen den Endoskopschaft (1) anpressen.

5. Endoskop gemäß Anspruch 1, **gekennzeichnet durch** eine weitere Schmiermittelförder-und Zuführeinrichtung (19) die an ein Gehäuse (11) der Antriebseinrichtung (10) angeschlossen ist, um das geförderte Schmiermittel in den dicht verschlossenen Innenraum des Gehäuses (11) zu pressen, wobei das Schmiermittel über die Antriebseinrichtung (10) in einem Hohlraum zwischen dem inneren und äußeren Schlauchabschnitt (2a, 2b, 2c) des Stülpschlauchs (2) gelangt.

## Claims

1. Endoscope with an everting tube system, and with an inner shaft (1) or a shaft-like component which is guided slidingly in a tube (2) that is everted at both axial end areas and can be moved by a drive mechanism (10) acting on an inner tube section (2a) of the everting tube (2), a lubricating device (15, 16, 17, 18) being provided for introducing lubricants into an annular gap (9) between the shaft (1) and the inner section (2a) of the everting tube, **characterized in that** for this purpose at least one substantially radial bore (17) or perforation, provided in the wall of the shaft (1), opens into the annular gap (9) and is connected to a lubricant-conveying device (15).

2. Endoscope according to Claim 1, **characterized in that** lubricant is delivered to the bore (17) or perforation via the cavity inside the shaft (1), which thus forms a section of a lubricant delivery line (16).

3. Endoscope according to Claim 1, **characterized in that** a delivery line (16), routed inside the shaft (1), is connected at one end to the bore (17) or perforation and is connected at its other end to an external lubricant line (18c), which opens into a pressurized container (18) for lubricant.

4. Endoscope according to one of the preceding claims, **characterized in that** the everting tube (2) has front and rear everting areas (2d, 2e) each taking the form of a bulge that narrows the internal diameter of the everting tube (2) in such a way that the everting areas (2d, 2e) press sealingly against the endoscope shaft (1).

5. Endoscope according to Claim 1, **characterized by** a further lubricant-conveying and delivering mechanism (19), which is connected to a housing (11) of the drive mechanism (10) in order to press the conveyed lubricant into the tightly sealed interior of the housing (11), the lubricant passing through the drive mechanism (10) into a cavity between the inner and outer tube sections (2a, 2b, 2c) of the everting tube (2).

## Revendications

1. Endoscope avec un système à manchon inversé et une tige interne (1), ou un élément identique à une tige, qui est conduite de façon coulissante dans un manchon (2) retroussé à chacune des deux extrémités axiales, qui est déplaçable grâce à un dispositif d'entraînement (10), qui agit sur une, un dispositif de lubrification (15, 16, 17, 18) étant prévu pour l'injection de lubrifiant dans une rainure annulaire (9) entre la tige (1) et dans la section interne de manchon inversé (2a) du manchon (2), **caractérisé en ce qu'**il est prévu un passage (17) ou perforation s'étendant essentiellement radialement dans la paroi de la tige, qui s'ouvre sur la rainure annulaire (9) et qui est relié à un dispositif de circulation de lubrifiant (15).

2. Endoscope selon la revendication 1; **caractérisé en ce que** l'alimentation en lubrifiant vers le passage (17) ou perforation se fait par l'intermédiaire de l'espace creux à l'intérieur de la tige (1) qui forme ainsi une partie de la conduite d'alimentation en lubrifiant (16).

3. Endoscope selon la revendication 1; **caractérisé en ce qu'**une conduite d'alimentation (16) est disposée à l'intérieur de la tige (1) reliée à l'une de ses extrémités au passage (17) ou perforation et à son autre extrémité à une conduite externe de lubrification (18c) qui débouche dans un réservoir sous pression de lubrifiant (18).

4. Endoscope selon l'une des revendications précédentes; **caractérisé en ce que** le manchon retroussé (2) a un secteur retroussé avant et arrière (2d, 2e) qui prennent chacun la forme d'un bourrelet rétrécissant le diamètre interne du manchon retroussé de telle façon que les secteurs retroussés (2d, 2e) se pressent de façon étanche contre la tige de l'endoscope (1).

5. Endoscope selon la revendication 1; **caractérisé en ce qu'**un dispositif de déplacement et d'alimentation en lubrifiant (19) qui est relié à un boîtier (11) du dispositif d'entraînement (10), pour presser le lubrifiant déplacé dans l'espace non clos du boîtier (11), le lubrifiant parvenant au moyen d'un du dispositif d'entraînement (10) dans un espace vide entre les secteurs de manchon interne et externe (2a, 2b, 2c) du manchon retroussé (2).
